# EUROPEAN PATENT SPECIFICATION

(11) **EP 3 542 764 B1**
(45) Date of publication and mention of the grant of the patent: **16.12.2020**
(21) Application number: 16921790.8
(22) Date of filing: 20.12.2016
(51) Int. Cl.: A42B 1/00, A42B 1/22, A42B 1/04, A61F 7/10

(54) **MEDICAL INNER CAP**
MEDIZINISCHE INNENKAPPE
BONNET INTÉRIEUR MÉDICAL

(30) Priority: 17.11.2016 JP 2016005534 U
(43) Date of publication of application: 25.09.2019
(73) Proprietor: Hair Clinic Reve-21 Corporation, Osaka-shi, Osaka 540-6122 (JP)
(72) Inventor: OKAMURA Katsumasa, Osaka-shi Osaka 540-6122 (JP)
(74) Representative: Horn Kleimann Waitzhofer Patentanwälte PartG mbB
(86) International application number: PCT/JP2016/087988
(87) International publication number: WO 2018/092323

(56) References cited:
- WO-A1-2011/099119
- GB-A- 519 347
- JP-A- S5 829 459
- JP-A- 2008 150 730
- JP-A- 2008 150 730
- JP-A- 2015 158 027
- JP-U- 3 025 709
- US-A- 1 991 055
- US-A- 2 698 945
- US-A1- 2003 131 861
- US-A1- 2015 128 979

## Description

### TECHNICAL FIELD

The present invention relates to medical caps, in particular, to a medical cap used as an inner cap for helmets employed in cooling the scalp.

### BACKGROUND ART

Chemotherapies used in cancer treatment are often accompanied by hair loss, wherein patients' psychological distress and other significant lowering of quality-of-life has been a problem. It is known that such hair loss can be prevented or suppressed by keeping the scalp at a low temperature to keep the hair roots from absorbing the therapeutic drugs for chemotherapy. For this reason, a head cooling device directed to preventing hair loss due to chemotherapy has been proposed (reference is made to JP 5133355 B2). With head cooling devices of this kind, a cooling-medium flow path is formed inside a helmet-shaped member that is fitted onto a user's head, and the cooling medium is made to circulate in the cooling-medium flow path to cool the scalp by heat exchange between the scalp and the cooling medium.

The helmet-shaped component that is fitted onto the head of the user is often manufactured by molding from a synthetic resin such as a silicone rubber. Although silicone rubbers have a certain degree of flexibility, since the shape and size of users' heads vary, it is difficult to bring the helmet-shaped component into close contact with the entire head. The inner surface of the helmet-shaped member functions as a heat exchange surface. If a gap arises between the user's scalp and the inner surface of the helmet-shaped component and the scalp, a portion of the scalp loses contact with the inner surface of the helmet-shaped component, leading to problems of the cooling of the scalp becoming uneven or the cooling efficiency deteriorating. As a method of reducing such a gap, in order to facilitate conforming to the shape and size of a user's head, fitting an inner element made of a flexible material between a cap-shaped member and the scalp is also conceivable. However, since the inner element is generally made of a material such as fiber or foam, and such a material has thermally insulating properties, the presence of the inner element interferes with heat conduction, and a problem that the cooling efficiency ends up being reduced arises instead.

Therein, in view of such problems, the applicant of the present application has proposed an inner cap that is fitted onto the inside of a cooling cap for cooling a user's head, the inner cap including a non-woven fabric layer formed of a nonwoven fabric, the nonwoven fabric layer being a water absorption layer made of a highly water-absorbent plastic fiber material (JP 2015-158027 A).

US 1991055A discloses a head covering helmet comprising an elongated, upwardly arched and transversely curved central section extending from to back, and side sections having curved edges secured to lateral edges of the central section by permanent stitchings. At least one of said sections includes two overlapped parts relatively adjustable to regulate the head size of the helmet.

### SUMMARY OF THE INVENTION

### PROBLEM TO BE SOLVED BY THE INVENTION

The inner cap presented by the applicant of the present application is provided a superior structure, but as a result of clinical trials, the scalp turned red and mild frostbite was occasionally observed in some users. That is, the known inner cap is believed to bring about overcooling to the scalp. Therein, an object of the invention is to propose a medical cap that is easy for the user to wear, has a suitable degree of heat-insulating effectiveness, and can be used as the inner cap of a helmet for a scalp-cooling device that cools the scalp.

### MEANS FOR SOLVING PROBLEM

In order to solve the above-mentioned problems, a medical cap of the invention has the features recited in claim 1. Further, the main body may be provided with a handle on an edge portion of the frontal region.

A hook-and-loop fastener is provided on a lining of the finlike protrusions on the left temporal piece and the right temporal piece, and may be formed to be connectable to hook-and-loop fasteners provided opposing each other across the cut on left and right shell fabric in the nape region, to fit a user's head dimensions.

### EFFECT OF THE INVENTION

The medical cap according to the invention can be adapted to persons with different head sizes by adjusting the size of the inner cap or selecting from several kinds of sizes, and a stable wearing state can be obtained. In addition, since a nonwoven or like fabric is used as the material, it is less likely to deteriorate, and there is also little concern of causing itching or rash on the human body. In addition, the medical cap may be made for washing and reuse, or may be made disposable.

### BRIEF DESCRIPTION OF DRAWINGS

Fig. 1 is a open-out view of a medical cap involving the invention;
Fig. 2 is a right-side view of the medical cap involving the invention;
Fig. 3(a) is a front view of the medical cap involving the invention and Fig. 3(b) is a rear view thereof and
Fig. 4 is a view illustrating a state in which a user has fitted on the medical cap involving the invention.

### MODE(S) FOR CARRYING OUT THE INVENTION

Hereinafter, embodiments of the invention will be described in detail with reference to the drawings. In each of the drawings, the same parts are denoted by the same reference numerals and repeated description will not be provided. It should also be noted that the drawings may be exaggerated for the purpose of understanding the invention and are not necessarily drawn to scale. Further, the invention is not limited to the examples illustrated below.

### Example 1

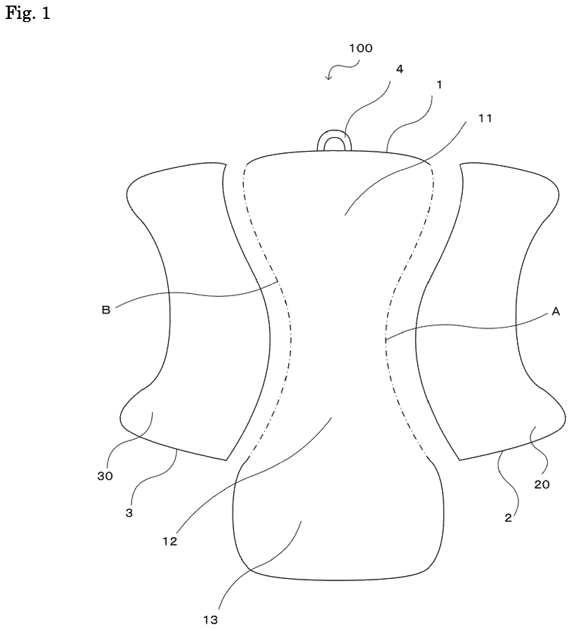

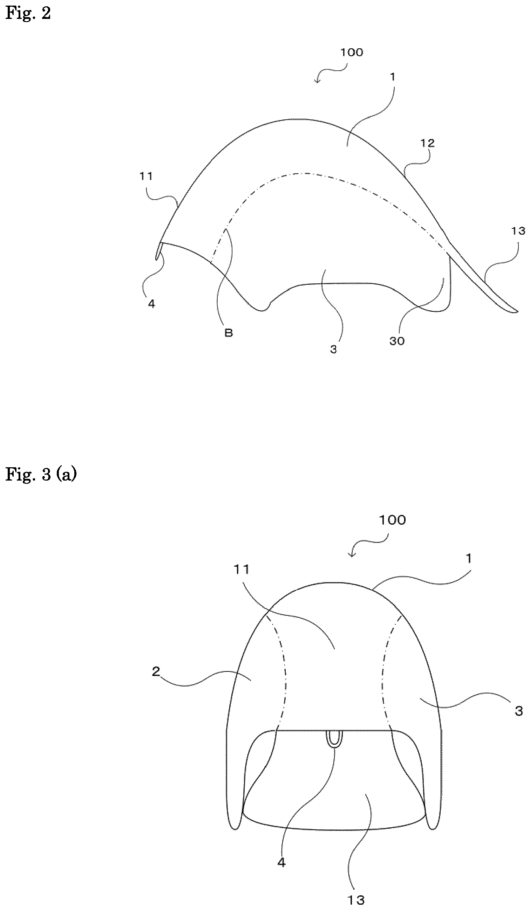

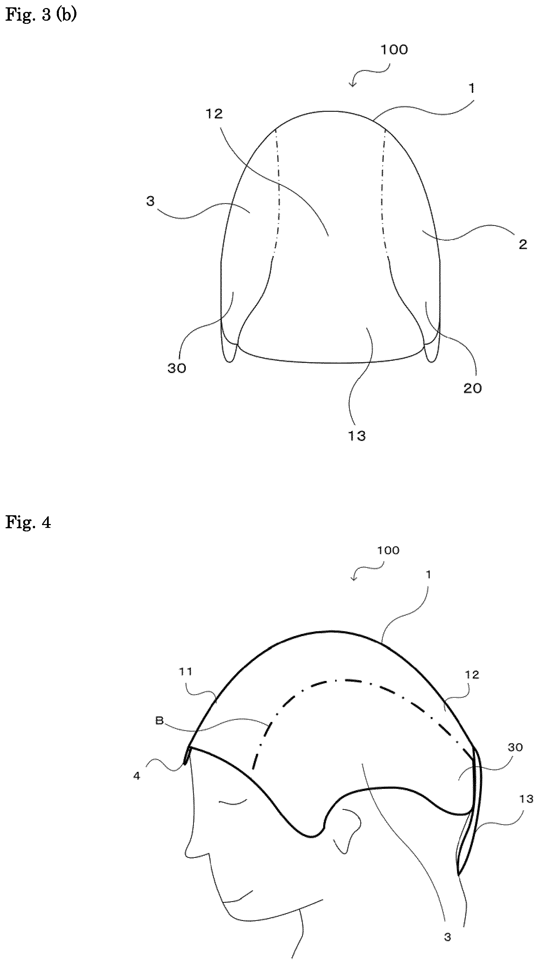

Example 1 will be described in detail with reference to the drawings. Fig. 1 is an opened-out view of a medical cap according to the invention. Fig. 2 is a right-side view of the medical cap according to the invention. Fig. 3(a) is a front view of the medical cap according to the invention, and Fig. 3(b) is a rear view thereof. Fig. 4 is a view illustrating a state in which user has fitted on a medical cap according to the invention. As illustrated in Figs. 1 to 4, a medical cap 100 according to Example 1 generally includes a main body 1, a left temporal piece 2, and a right temporal piece 3.

Here, referring to Fig. 1. The main body 1 includes a frontal region 11, an occipital region 12, and a nape region 13. The main body 1 is integrally sewn with the left temporal piece 2 at a sewn part A (indicated by alternating long and short dashed lines), and is integrally sewn with the right temporal piece 3 at a sewn part B (indicated by alternating long and short dashed lines). In addition, connection parts between the main body 1 and the left temporal piece 2 and between the main body 1 and the right temporal piece 3 are not restricted to sewing. The left temporal piece 2 and the right temporal piece 3 respectively form finlike protrusions 20 and 30 at the rear end, and a cut is formed between the finlike protrusions 20 and 30 and the nape region 13.

As illustrated in Fig. 1, the main body 1 may have a handle 4 attached to the front end, that is, an edge portion of the frontal region 11. When a user wears the medical cap 100 and fits on the cooling helmet for a scalp-cooling device, the user can correct displacement of the medical cap 100 by pulling the handle 4.

In addition, the fabric body used for the medical cap 100 may have a suitable degree of heat-insulating effectiveness. That is, any material that can be processed into the shape of the medical cap 100 illustrated in Figs. 1 to 4 may be used. For example, it is possible to select chemical fibers such as polyester, cotton, nonwoven fabric, and paper as the fabric body, silk, plastics such as thin cloth, and the like as the other material.

Here, referring to Fig. 2. Fig. 2 is a right-side view of the medical cap 100 according to the invention. The right temporal piece 3 is sewn to the main body 1 at the upper end. The right temporal piece 3 has the finlike protrusion 30 formed at the rear end, and a cut is formed between the finlike protrusion 30 and the nape region 13. A lower line of the right temporal member 3 may be concavely curved so as not to cover the ear. Although not illustrated, a hook-and-loop fastener is provided on the lining of the finlike protrusion 30 of the right temporal piece 3, and may be formed to be connectable to a hook-and-loop fastener provided in the nape region 13 on shell fabric facing across the cut, to fit the head dimensions of a user. Since the left lateral side is the same as the right lateral side, the description thereof will not be provided.

Here, reference is made to Fig. 3. Fig. 3(a) is a front view of the medical cap 100 according to the invention, and Fig. 3(b) is a rear view thereof. As illustrated in Fig. 3(a), the handle 4 may be attached to the front end of the main body 1, that is, the edge portion of the frontal region 11. The handle 4 may be any member capable of being gripped and pulled by the user, and may be a loop-like member. Alternatively, a tongue piece member may be sewn to the front end of the frontal region 11.

Here, reference is made to Fig. 4. Fig. 4 is a view illustrating a state in which the user has fitted on a medical cap 100 according to the invention. As illustrated in Fig. 4, it will be appreciated that the medical cap 100 according to the invention is a medical cap made up of a fabric body sewn to cover the head of the user, and includes a frontal region 11 that covers the forehead including the brow, the occipital region 12 that covers the occipital, the main body 1 including the nape region 13 covering the nape, and the right temporal piece 3 connected thereto, the right temporal piece 3 connected to the main body 1 forms the finlike protrusion 30 at the rear end, and a cut is formed between the finlike protrusion 30 and the nape region 13. It is possible to adapt the medical cap to people with different head sizes by such a cut, and a stable wearing condition can be obtained.

The medical cap 100 of the invention is suitable as an inner cap of a scalp-cooling-device helmet for cooling the scalp, since it has a suitable degree of heat-insulating effectiveness, and when the helmet is fitted on with the inner cap being worn, since the inner cap serves as a cushioning material between the helmet and the scalp, a comfortable fit is provided for the user.

While the preferred embodiment of the medical cap according to the invention has been illustrated and described, it will be understood that various modifications can be made without departing from the scope of the invention.

### INDUSTRIAL APPLICABILITY

A medical cap involving the invention finds broad utility not only as an inner cap of a helmet for a scalp cooling device, but also as a hat that covers the head in cases where a patient has lost hair due to the side effects of anticancer drugs.

### EXPLANATIONS OF LETTERS OR NUMERALS

:
- 100: MEDICAL CAP

- 1: MAIN BODY
- 11: FRONTAL REGION
- 12: OCCIPITAL REGION
- 13: NAPE REGION
- 2: LEFT TEMPORAL PIECE
- 3: RIGHT TEMPORAL PIECE
- 20, 30: FIN-LIKE PROTRUSION
- 4: HANDLE

## Claims

1. A medical cap (100) to be used as an inner cap of a helmet for a scalp cooling device for cooling the human scalp and made up of a fabric body formed to cover a human head, the medical cap (100) comprising:
a main body (1) including:
a frontal region (11) for covering the forehead including the brow,
an occipital region (12) for covering the occipital, and
a nape region (13) for covering the nape, and
a left temporal piece (2) and a right temporal piece (3) connected to the main body (1); **characterized in that**
the left temporal piece (2) and the right temporal piece (3) are connected to the main body (1) respectively to form rear end finlike protrusions (20, 30), and cuts are formed between the rear end finlike protrusions (20, 30) and the nape region (13), and
a hook-and-loop fastener is provided on a lining of the rear end portions (20, 30) on the left temporal piece (2) and the right temporal piece (3), and is formed to be connectable to hook-and-loop fasteners provided opposing each other across the cut on left and right shell fabric in the nape region (13), to fit a user's head dimensions.

2. A medical cap (100) according to claim 1, wherein the main body (1) is provided with a handle on an edge portion of the frontal region (11).

3. A medical cap (100) according to claim 1 or 2, wherein a lower line of the temporal pieces (2, 3) is concavely curved so as not to cover the ear.

## Patentansprüche

1. Medizinische Kappe (100), die als eine Innenkappe eines Helms für eine Kopfhautkühlvorrichtung zum Kühlen der menschlichen Kopfhaut verwendbar ist und aus einem Stoffkörper besteht, der so geformt ist, dass er einen menschlichen Kopf bedeckt, wobei die medizinische Kappe (100) umfasst:
einen Hauptkörper (1), umfassend:
einen Frontalbereich (11) zum Bedecken des Vorderkopfs einschließlich der Stirn,
einen Hinterkopfbereich (12) zur Abdeckung des Hinterhauptbeins, und
einen Nackenbereich (13) zur Abdeckung des Nackens, und
ein linkes Temporalstück (2) und ein rechtes Temporalstück (3), die mit dem Hauptkörper (1) verbunden sind; **dadurch gekennzeichnet, dass**
das linke Temporalstück (2) und das rechte Temporalstück (3) jeweils mit dem Hauptkörper (1) verbunden sind, um hintere flossenartige Vorsprünge (20, 30) zu bilden, und Schnitte zwischen den hinteren flossenartigen Vorsprüngen (20, 30) und dem Nackenbereich (13) gebildet sind, und
ein Klettverschluss an einer Auskleidung der hinteren Endabschnitte (20, 30) am linken Temporalstück (2) und am rechten Temporalstück (3) vorgesehen ist und so ausgebildet ist, dass er mit Klettverschlüssen verbindbar ist, die einander gegenüberliegend jenseits des Schnitts am linken und rechten Schalenstoff im Nackenbereich (13) vorgesehen sind, um sich an die Kopfabmessungen eines Benutzers anzupassen.

2. Medizinische Kappe (100) nach Anspruch 1, wobei der Hauptkörper (1) mit einem Griff an einem Randabschnitt des Frontalbereichs (11) versehen ist.

3. Medizinische Kappe (100) nach Anspruch 1 oder 2, wobei eine untere Linie der Temporalstücke (2, 3) konkav gekrümmt ist, so dass sie das Ohr nicht bedeckt.

## Revendications

1. Bonnet médical (100) destiné à être utilisé comme bonnet intérieur d'un casque pour un dispositif de refroidissement du cuir chevelu humain et constituée d'un corps en tissu formé pour couvrir une tête humaine, le bonnet médical (100) comprenant :
un corps principal (1) comprenant :
une région frontale (11) destinée à couvrir l'avant de la tête, y compris le front,
une région occipitale (12) pour couvrir l'occipital, et
une région de la nuque (13) pour couvrir la nuque, et
une pièce temporale gauche (2) et une pièce temporale droite (3) reliées au corps principal (1) ; **caractérisé en ce que**
la pièce temporale gauche (2) et la pièce temporale droite (3) sont respectivement reliées au corps principal (1) pour former des saillies arrière en forme d'ailettes (20, 30), et des entailles sont formées entre les saillies arrière en forme d'ailettes (20, 30) et la région de la nuque (13), et
une fermeture scratch est prévue sur un revêtement des parties d'extrémité arrière (20, 30) sur la pièce temporale gauche (2) et la pièce temporale droite (3), et est formée de manière à pouvoir être reliée à des fermetures scratch prévues en opposition l'une par rapport de l'autre coté de l'entaille sur des coques de tissu gauche et droite dans la région de la nuque (13), pour s'adapter aux dimensions de la tête de l'utilisateur.

2. Bonnet médical (100) selon la revendication 1, dans lequel le corps principal (1) est muni d'une poignée sur une partie de bord de la région frontale (11).

3. Bonnet médical (100) selon la revendication 1 ou 2, dans lequel une ligne inférieure des pièces temporales (2, 3) est incurvée de manière concave afin de ne pas couvrir l'oreille.
